# EUROPÄISCHE PATENTANMELDUNG

(11) **EP 0 873 729 A2**
(43) Veröffentlichungstag der Anmeldung: **28.10.1998**
(21) Anmeldenummer: 98106121.1
(22) Anmeldetag: 03.04.1998
(51) Int. Cl.: A61F 2/06

(54) **Radial aufweitbare Stützvorrichtung III**

(30) Priorität: 25.04.1997 DE 19717477
(71) Anmelder: W.C. Heraeus GmbH, D-63450 Hanau (DE)
(72) Erfinder: Gorywoda, Marek Dr., 63452 Hanau (DE)
(74) Vertreter: Kühn, Hans-Christian

(57) **Zusammenfassung**

Die Erfindung betrifft eine radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, die im wesentlichen in Längsrichtung des rohrförmigen Körpers verlaufen, wobei jeweils zwei benachbarte Glieder unter Bildung eines einen Schlitz bildenden Gliederpaares an ihren Enden miteinander verbunden sind und wobei die Gliederpaare untereinander mit, jeweils in Umfangsrichtung des rohrförmigen Körpers gesehen, benachbarten Gliederpaaren verbunden sind.

Um während der Aufweitung der Stützstruktur keine oder nur eine sehr geringe Längenverkürzung zu erhalten, sind benachbarte Gliederpaare jeweils durch einen Steg miteinander verbunden, wobei die geometrischen Mittelpunkte von mindestens zwei dieser benachbarten Gliederpaare in Längsrichtung des rohrförmigen Körpers gegeneinander versetzt sind.

## Beschreibung

Die Erfindung betrifft eine radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, die im wesentlichen in Längsrichtung des rohrförmigen Körpers verlaufen, wobei jeweils zwei benachbarte Glieder unter Bildung eines einen Schlitz bildenden Gliederpaares an ihren Enden miteinander verbunden sind und wobei die Gliederpaare untereinander mit, jeweils in Umfangsrichtung des rohrtörmigen Körpers gesehen, benachbarten Gliederpaaren verbunden sind.

Derartige Stützstrukturen sind beispielsweise aus EP 221 570 B1 oder aus EP 335 341 B1 bekannt. Hier sind Stützstrukturen beschrieben, die aus langgestreckten Gliederpaaren gebildet sind. Diese Stützstrukturen werden in verengte Blutgefäße oder andere ein Lumen aufweisende Körperdurchgänge eingeschoben, um diese nach Erweiterung durch eine Ballondilatation offen zu halten. Dabei werden die Stützstrukturen in ihrem Durchmesser aufgeweitet und sie verkürzen sich während der Aufweitung. Derartige Verkürzungen sind allerdings in der Regel unerwünscht, da diese Verkürzung dazu führt, daß eine wesentlich längere Stütrstruktur in die Körperöffnung eingeführt werden muß, als sie am Einsatzort unmittelbar erforderlich ist. Die bekannten Strukturen passen sich Bögen oder Kurven in den Körperöffnungen relativ schlecht oder gar nicht an, so daß zusätzliche Biegungselemente vorgesehen werden müssen (EP 335 341 B1). Die bekannten Stützstrukturen weisen starre rohrförmige Abschnitte auf, die durch gelenkige Verbindungen biegbar miteinander verbunden sind. In der Praxis hat es sich gezeigt, daß in diesen gelenkigen Bereichen, bedingt durch die Dauerunruhe im Gewebelager, Gewebshypertrophien entstehen können.

Andere bekannte Strukturen weisen eine ausgeprägte Verkürzung bei der Dehnung auf. Auch spiralige Strukturen sind bekannt. Diese haben allerdings ein für den Einsatz ungünstiges Verhalten an ihren Enden.

Aufgabe der vorliegenden Erfindung ist es, eine radial aufweitbare Stützstruktur zu schaffen, die während ihrer Aufweitung keine oder nur eine sehr geringe Längenverkürzung erfährt.

Die Aufgabe wird für die eingangs genannte Stützstruktur dadurch gelöst, daß benachbarte Gliederpaare jeweils durch einen Steg miteinander verbunden sind und daß die geometrischen Mittelpunkte von mindestens zwei dieser benachbarten Gliederpaare in Längsrichtung des rohrförmigen Körpers gegeneinander versetzt sind. Derart benachbarte Gliederpaare sind in ihrer Länge begrenzt. Die Gliederpaare als solche weiten sich bei Dehnung auf, daß heißt, die durch die Gliederpaare gebildeten Schlitze werden breiter. Durch die in Längsrichtung begrenzte Ausdehnung der Schlitze ist auch die Verkürzung der gesamten Struktur bei Ausdehnung nur sehr gering. Die erfindungsgemäße Struktur weist um ihren Umfang herum verteilt mehrfach eine, in Längsrichtung der Stützstruktur gesehen, eine Hintereinanderanordnung von Gliederpaaren auf, wobei in Umfangsrichtung gesehen benachbarte Gliederpaare sich hinsichtlich ihrer Enden überlappen. Dadurch wird nicht nur die Verkürzung bei Ausdehnung begrenzt, sondern die Struktur ist in sich flexibel, kann also auf spezielle Biegungselemente oder Gelenke verzichten. Die in Längsrichtung des rohrförmigen Körpers hintereinander angeordneten Enden von Gliederpaaren sind vorzugsweise nicht unmittelbar miteinander verbunden, so daß eine Verformung dieser Gliederpaare von einander entkoppelt ist. Die Enden der Gliederpaare können durch eine Rundung oder durch rechtwinklig zwischen den benachbarten Gliedern angeordnete Verbindungsstücke miteinander verbunden sein. Die Glieder sind zwar erfindungsgemäß langgestreckt, sie müssen jedoch nicht geradlinig verlaufen, beispielsweise können sie auch in einem oder mehreren Bögen geschwungen sein, im Zickzack verlaufen oder spiralförmig oder in anderer Form. Die Ausdehnung in Längsrichtung der Stützstruktur sollte größer sein als Amplitude der Form (in Umfangsrichtung gesehen). Derartig geformte Glieder bzw. Abschnitte dieser Glieder (eine abschnittsweise Verformung ist vorzugsweise auch möglich) wirken als Dehnungsausgleichsabschnitte, die bei einer Dehnung zusätzlich eine Verkürzung der Längsausdehnung minimieren bzw. auch ganz unterdrücken können. Vorteilhafterweise sind diese Dehnungsausgleichsabschnitte in der Mitte der Schlitze angeordnet bzw. symmetrisch zur Mitte der Schlitze (in Längsrichtung gesehen). Prinzipiell können die langgesstreckten Glieder auch paarweise in Längsrichtung sich erstreckende Ovale oder Rautenstrukturen bilden. Die Stützstruktur kann eine biokompatible Beschichtung aufweisen, wie sie beispielsweise aus EP 335 341 B1 bekannt ist. Die rohrförmigen Körper sind aus nahtlosen Rohren gebildet, um Verspannungen zu vermeiden. Die Strukturen (Anordnung der Glieder) sind durch Laserschneiden, Elektroerosion, Ätzen oder spanabhebend hergestellt.

Weitere vorteilhafte Ausgestaltungen sind in den Unteransprüchen angegeben.

Nachfolgend werden Ausführungsbeispiele der erfindungsgemäßen Stützstruktur anhand einer Zeichnung erläutert. In der Zeichnung zeigt
- Figur 1: die schematische Darstellung einer in eine Ebene abgerollten Wandfläche der Stützstruktur mit rechteckig ausgebildeten Schlitzen,
- Figur 2: eine kürzere Form einer solchen Stützstruktur nach Figur 1,
- Figur 3: ebenfalls eine kürzere Struktur gemäß Figur 1,
- Figur 4: eine Stützstruktur, bei der die langgestreckten Glieder durch eine Rundung an ihren Enden zu Gliederpaaren verbunden sind,
- Figur 5: eine Stützstruktur mit winklig ausgebildeten langgestreckten Gliedern und
- Figur 6: eine Stützstruktur mit ovalen Gliederpaaren.

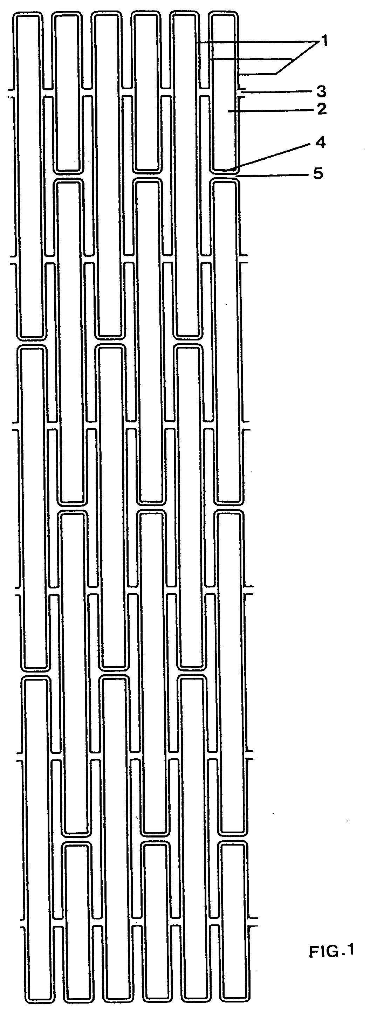

Figur 1 zeigt die abgerollte Wandfläche einer Stützvorrichtung. Die Vorrichtung ist aus einem für medizinische Zwecke geeigneten Material, beispielsweise einem Edelstahl gebildet. Die sich geradlinig in Längsrichtung erstreckenden Glieder 1 sind paarweise zu Gliederpaaren verbunden, die einen Schlitz 2 bilden. Diese Gliederpaare wirken als Dehnungsglieder, die sich in Umfangsrichtung aufweiten, wenn die Stützvorrichtung innerhalb eines Körpergefäßes oder einer Körperöffnung aufgeweitet wird. Die Glieder 1 der Gliederpaare werden an ihren Enden durch Verbindungsstücke 4 miteinander verbunden. In Längsrichtung der Stützvorrichtung sind mehrere derartige Gliederpaare hintereinander angeordnet, wobei die einzelnen Gliederpaare voneinander getrennt sind, so daß sich eine flexible Struktur ergibt und die Längenverkürzung bei radialer Ausdehnung der Stützvorrichtung minimiert wird. In Umfangsrichtung nebeneinander angeordnete Gliederpaare (im gezeigten Beispiel sind es sechs Stück) sind zueinander versetzt, wobei die Trennstellen 5, die zwischen in Längsrichtung hintereinander angeordneten Gliederpaaren vorhanden sind, in Umfangsrichtung derart angeordnet sind, daß sie sich jeweils in der Mitte (in Längsrichtung gesehen) der unmittelbar benachbarten Gliederpaare befinden. Diese benachbarten Gliederpaare sind also überlappend angeordnet. Dabei besteht zwischen zwei derartig benachbarten Gliederpaaren jeweils eine Verbindung durch einen Steg 3 und jedes Gliederpaar ist auf jeder Seite mit jeweils zwei benachbarten Gliederpaaren durch einen solchen Steg 3 verbunden. Ausnahme hiervon stellen die Glieder 1 an den Enden der Stützvorrichtung dar. Hier ist jedes zweite Gliederpaar lediglich halb so lang wie die benachbarten Gliederpaare und auch nur mit einem solcher benachbarten Gliederpaare an jeder Seite verbunden.

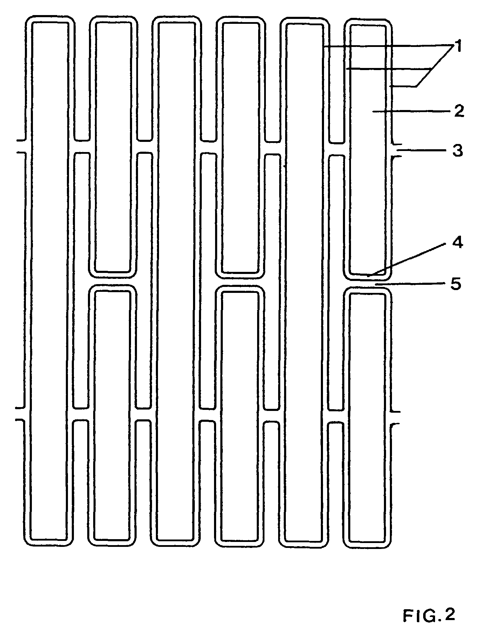

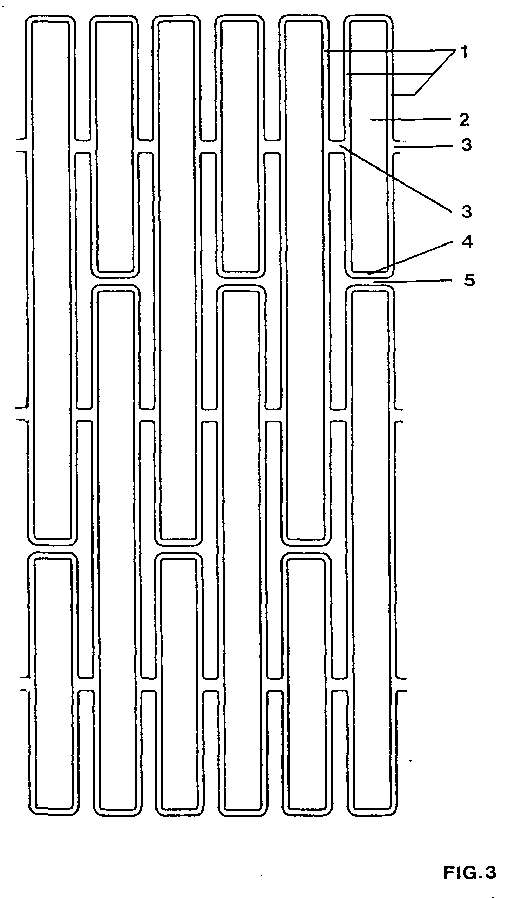

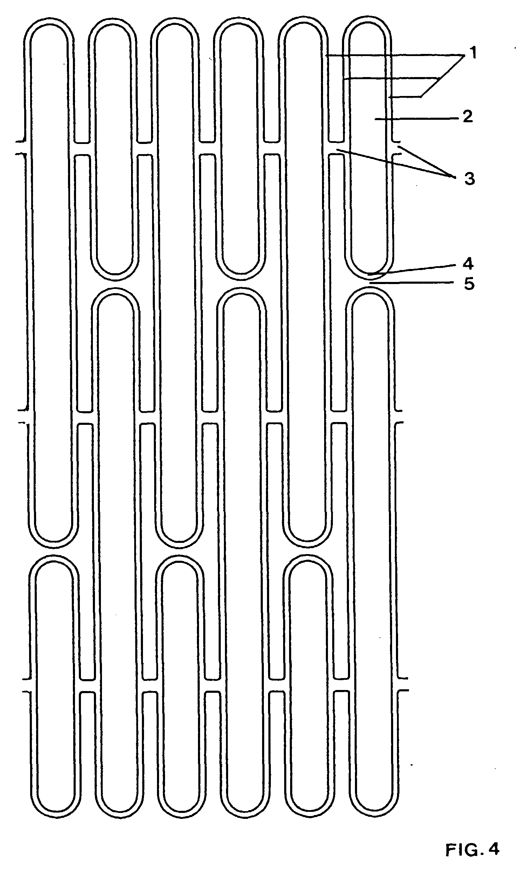

In Figur 2 ist eine sehr kurze Stützvorrichtung dargestellt, die, in Umfangsrichtung gesehen, derart aufgebaut ist, daß im Wechsel jeweils ein langes Gliederpaar, daß sich über die gesamte Länge der Stützvorrichtung erstreckt und zwei kurze Gliederpaare angeordnet sind. Eine weitere Struktur ist in Figur 3 dargestellt. Diese Struktur ist von mittlerer Länge, wobei in Längsrichtung hintereinander ein langes Gliederpaar und ein kurzes Gliederpaar angeordnet sind. Figur 4 zeigt eine ähnliche Anordnung, wobei die Stege 4 bogenförmig ausgebildet sind und die jeweils benachbarten, ein Gliederpaar bildenden Glieder 1 durch stetigen Übergang mit den Stegen 4 verbunden sind.

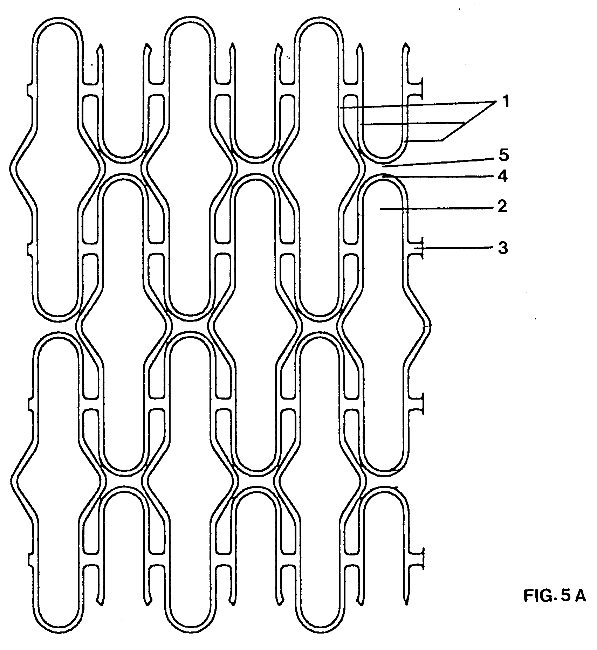

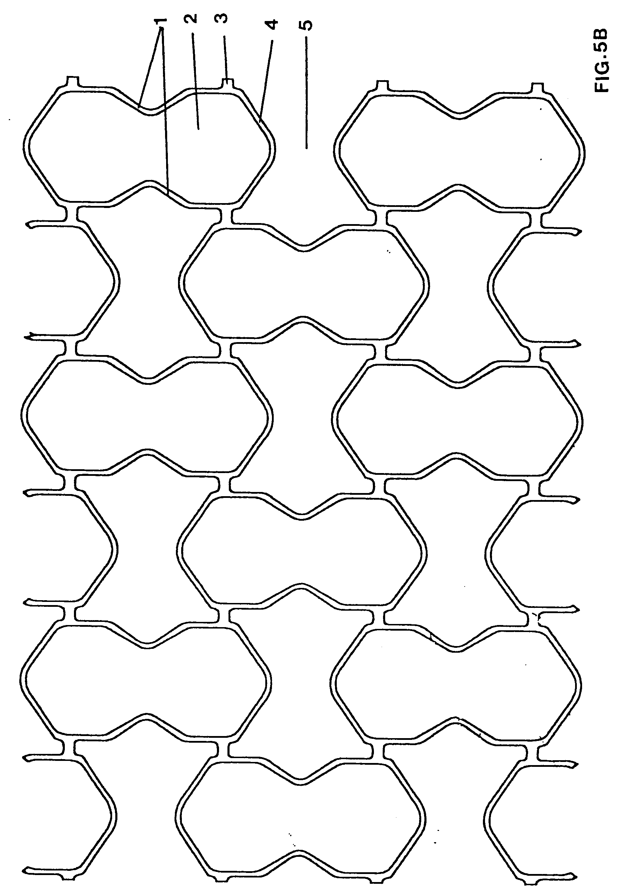

Figur 5 zeigt eine weitere Ausführungsform, die prinzipiell genauso aufgebaut ist wie die in den Figuren 1 bis 4 gezeigten Darstellungen. Das besondere dieser Ausführung besteht im wesentlichen darin, daß die Glieder 1 in ihrem Mittelabschnitt als Dehnungsausgleichsabschnitt eine Aufweitung (Figur 5 a) oder eine Einengung (Figur 5 b) des Schlitzes ausbilden. Die Stege 4 bilden dabei entweder abgerundete Übergänge (Figur 5 a) oder sie sind im wesentlichen geradlinig ausgebildet und weisen in ihrer Mitte einen Knick auf (Figur 5 b).

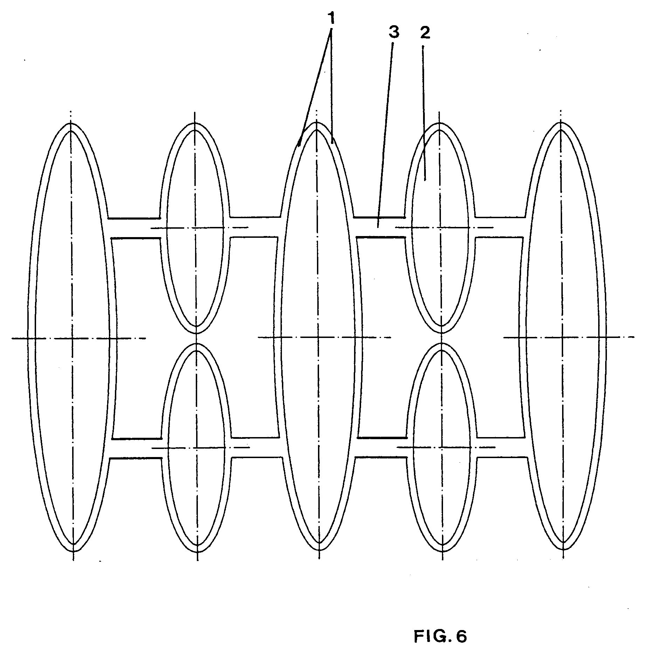

Figur 6 zeigt eine Anordnung ähnlich Figur 2, wobei die Gliederpaare durch gekrümmte Glieder gebildet sind, die an ihren Enden miteinander verbunden sind, so daß sich ovale Schlitze ergeben.

## Patentansprüche

1. Radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, die im wesentlichen in Längsrichtung des rohrförmigen Körpers verlaufen, wobei jeweils zwei benachbarte Glieder unter Bildung eines einen Schlitz bildenden Gliederpaares an ihren Enden miteinander verbunden sind und wobei die Gliederpaare untereinander mit, jeweils in Umfangsrichtung des rohrförmigen Körpers gesehen, benachbarten Gliederpaaren verbunden sind, dadurch gekennzeichnet, daß derart benachbarte Gliederpaare jeweils durch einen Steg (3) miteinander verbunden sind und daß die geometrischen Mittelpunkte von mindestens zwei dieser benachbarten Gliederpaare in Längsrichtung des rohrförmigen Körpers gegeneinander versetzt sind.

2. Radial aufweitbare Stützstruktur nach Anspruch 1, dadurch gekennzeichnet, daß in Längsrichtung des rohrförmigen Körpers hintereinander angeordnete Enden von Gliederpaaren nicht unmittelbar miteinander verbunden sind.

3. Radial aufweitbare Stützstruktur nach Anspruch 1 oder 2, dadurch gekennzeichnet, daß die Enden der Gliederpaare durch eine Rundung oder durch rechtwinklig zwischen den benachbarten Gliedern (1) angeordnete Verbindungsstücke (4) miteinander verbunden sind.

4. Radial aufweitbare Stützstruktur nach einem der Ansprüche 1 bis 3, dadurch gekennzeichnet, daß die langgestreckten Glieder (1) mindestens teilweise geschwungen ausgebildet sind.

5. Radial aufweitbare Stützstruktur nach einem der Ansprüche 1 bis 4, dadurch gekennzeichnet, daß die langgestreckten Glieder (1) Dehnungsausgleichsabschnitte aufweisen.

6. Radial aufweitbare Stützstruktur nach Anspruch 5, dadurch gekennzeichnet, daß die Dehnungsausgleichsabschnitte bogenförmig, als Kreisabschnitt, Zickzackstruktur oder spiralförmig ausgebildet sind.

7. Radial aufweitbare Stützstruktur nach Anspruch 5, dadurch gekennzeichnet, daß die Dehnungsausgleichsabschnitte in Längsrichtung des rohrförmigen Körpers gesehen etwa symmetrisch zur Mitte der Schlitze (2) angeordnet sind.

8. Radial aufweitbare Stützstruktur nach einem der Ansprüche 1 bis 7, dadurch gekennzeichnet, daß um den Umfang des rohrförmigen Körpers herum mindestens vier einen Schlitz bildende Gliederpaare benachbart zueinander angeordnet sind.

9. Radial aufweitbare Stützstruktur nach einem der Ansprüche 1 bis 8, dadurch gekennzeichnet, daß sie im wesentlichen aus einem oder mehreren Metalle der Gruppe Tantal, Titan, Niob, Stahl, Platin oder einer Legierung mindestens eines dieser Metalle mit mindestens einem weiteren Metall gebildet ist.

10. Radial aufweitbare Stützstruktur nach einem der Ansprüche 1 bis 9, dadurch gekennzeichnet, daß sie mit einem biokompatiblen Material beschichtet ist.
